# EUROPEAN PATENT APPLICATION

(11) **EP 1 790 384 A1**
(43) Date of publication of application: **30.05.2007**
(21) Application number: 06124367.1
(22) Date of filing: 20.11.2006
(51) Int. Cl.: A61N 7/00, A61B 8/00, A61M 37/00

(54) **Contrast agent augmented ultrasound therapy system with ultrasound imaging guidance for thrombus treatment**

(30) Priority: 23.11.2005 US 286983
(71) Applicant: Siemens Medical Solutions USA, Inc., Malvern, PA 19355 (US)
(72) Inventor: Cai, Anming He, San Jose, CA 95129 (US); Thomas, Lewis J, Palo Alto, CA 94306 (US)
(74) Representative: Hazzard, Alan David

(57) **Abstract**

Ultrasound diagnosis and therapy for disruption of a thrombus are provided with a same ultrasound system. A same transducer (14) and system images (30, 34) contrast agents and ruptures (38) the contrast agents to mechanically weaken or destroy a thrombus.

## Description

### BACKGROUND

The present embodiments relate to contrast agent ultrasound imaging augmented with ultrasound therapy.

Diagnosis with ultrasound imaging may use contrast agents. In low mechanical index (MI) imaging, the contrast agents within a region are imaged with minimal destruction. The flow or movement of the contrast agents may be monitored. Ultrasound may destroy contrast agents. Loss of correlation or other detection techniques may be used with some destruction of contrast agents. The destruction may enhance the imaging. Contrast agents are also destroyed for perfusion studies. Ultrasound energy ruptures the contrast agents to clear a region of the contrast agents. The wash-in of contrast agents is then monitored.

Therapy may be performed with ultrasound. Acoustic energy may heat a region. Drugs may be released from contrast agents by acoustic rupturing of the contrast agents. In another therapy, blood clots (thrombosis) may be broken or disrupted by acoustic energy.

Typically, diagnosis and therapy are performed with different systems. However, a same system and corresponding transducer may be used for both diagnosis and therapy. U.S. Patent No. 6,716,168, the disclosure of which is incorporated herein by reference, uses a same system and transducer to image and to stimulate drug uptake through heat generation.

### BRIEF SUMMARY

By way of introduction, the preferred embodiments described below include methods, instructions and systems for ultrasound imaging and therapy. A same transducer and system images contrast agents and drives the contrast agents to disrupt mechanically a thrombus.

In a first aspect, a method is provided for contrast agent ultrasound imaging and therapy. A possible clot and contrast agents are imaged with an ultrasound transducer. The contrast agents at or adjacent to the possible clot are driven with ultrasound pulses from the same ultrasound transducer.

In a second aspect, a computer readable storage medium has stored therein data representing instructions executable by a programmed processor for contrast agent ultrasound imaging and therapy. The instructions are for using one transducer to image a blood clot and contrast agents and to mechanically break or weaken the blood clot with disruption by at least some of the contrast agents.

In a third aspect, a method is provided for contrast agent ultrasound imaging and therapy. Acoustic energy is transmitted with an ultrasound transducer to a thrombosis. The transmission is about 0.5 MI or less. The ultrasound transducer is used to receive acoustic energy in response to the transmission. The received acoustic energy is responsive to contrast agents and the thrombosis. Additional acoustic energy is transmitted with the ultrasound transducer. The transmission is about 1.0 MI or more and operable to alter the contrast agents associated with the thrombosis.

In a fourth aspect, a method is provided for contrast agent ultrasound imaging and therapy in conjunction with thrombolytic agent. A possible clot and contrast agents are imaged with an ultrasound transducer. The contrast agents at or adjacent to the possible clot are driven with ultrasound pulse from the same ultrasound transducer.

The present invention is defined by the following claims, and nothing in this section should be taken as a limitation on those claims. Further aspects and advantages of the invention are discussed below in conjunction with the preferred embodiments and may be later claimed in independently or in combination.

### BRIEF DESCRIPTION OF THE DRAWINGS

The components and the figures are not necessarily to scale, emphasis instead being placed upon illustrating the principles of the invention. Moreover, in the figures, like reference numerals designate corresponding parts throughout the different views.

Figure 1 is a block diagram of one embodiment of a system for ultrasound imaging and therapy of a thrombus; and

Figure 2 is a flow chart diagram of one embodiment of a method for contrast agent ultrasound imaging and therapy with a same transducer.

### DETAILED DESCRIPTION OF THE DRAWINGS AND PRESENTLY PREFERRED EMBODIMENTS

One transducer is used for imaging a thrombus and contrast agents with or without drugs. The same transducer is used to drive the contrast agents for breaking the thrombus. The imaging and therapy are repeated as desired to disrupt sufficiently the thrombus.

In one example, low frequency (<2MHz) diagnostic ultrasound transmit pulses within the FDA MI limit of 1.9 break micro-bubbles with enough force to break the thrombus. The combination of low MI micro-bubble detection techniques in conjunction of low-frequency therapeutic transmit pulses may provide effective treatment of a thrombus without clot dissolving drugs that can cause significant side effects such as bleeding. Using the same transducer and system simplifies workflow for the operator as compared to separate therapy and imaging systems.

The ultrasound system responsible for generating the therapeutic ultrasound also generates an image of a thrombus. The same transmitter and transducer are used for generating B-mode, color Doppler, acoustic radiation force impulse imaging (ARFI), or other imaging and for applying acoustic therapy. The transmitter and/or transducer transmit both imaging pulses and therapeutic pulses. For example, a single linear transducer array with element spacing designed for imaging is also used for therapeutic ultrasound.

In one embodiment, a standard ultrasound system, such as the Antares^{™} or Sequoia^{®} System manufactured by Siemens Medical Solutions USA, Inc. Ultrasound Group, is used with little or no modification. The ultrasound system is capable of generating therapeutic pulses for each of the channels or transducer elements. Since contrast agent disruption is relied on for the therapy, acoustic energy within mechanical index and thermal limitations may be used. Using a standard or modified transducer, the system also generates images by transmission and reception of acoustic energy. The imaging pulses and therapeutic pulses are interleaved and provided from the same transducer.

By imaging and applying therapeutic ultrasound with the same transducer, more directed application of therapeutic ultrasound is provided. A field of view is imaged and a region of interest within the field of view is selected for therapeutic ultrasound. For example, a thrombus area is identified by imaging. The availability of contrast agents in or near the thrombus area is also identified by imaging. Therapeutic ultrasound energy is then transmitted to disrupt the contrast agents at the region of interest.

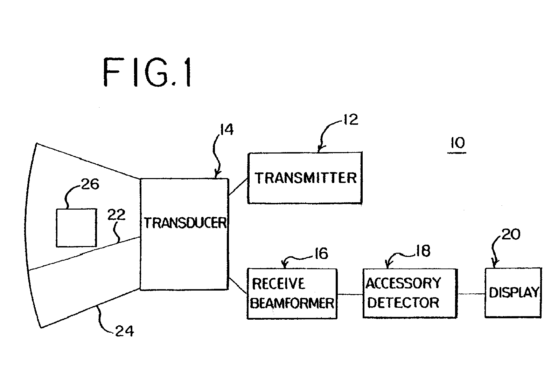

FIG. 1 shows an ultrasound system 10 for contrast agent therapy and imaging using ultrasound energy. The system 10 includes a transmit beamformer 12, a transducer 14, a receive beamformer 16, a processor or detector 18, and a display 20 electrically connected as shown. Additional, different or fewer components may be provided for the system 10. In one embodiment, the system 10 comprises a commercial ultrasound system from one of the manufactures listed above or another manufacturer.

The transducer 14 comprises a piezoelectric or a capacitive microelectromechanical ultrasound transducer. The transducer 14 has one or more elements for transducing between electrical and acoustical energies. In one embodiment, the transducer 14 includes only a single linear array of elements, such as a flat linear array or a curved linear array. In other embodiments, the transducer comprises a two-dimensional array, a 1.5 dimensional array or other multi-dimensional configurations of elements. The array of elements is configured for insertion into a patient or use external to a patient with or without mechanical rotation or position tracking devices.

The transducer 14 is a standard imaging transducer, such as a transducer associated with half wavelength spacing of elements sandwiched between a backing block for absorbing acoustic energy and matching layers for matching the acoustic impedance of the elements to a patient. For example, the transducer is a 4C1 probe available from Siemens Medical Solutions, USA.

In alternative embodiments, the transducer 14 is modified for heat dissipation. For example, a copper foil or copper braid is connected with a lens of the transducer 14 for dissipating heat from the lens. Different piezoelectric materials or matching layers may be optimized for providing a better acoustic or electrical impedance match, reducing an amount of heat generated by the transducer. In one embodiment, multiple layers of piezoelectric or microelectromechanical material separated by electrodes are provided for each element. The multiple layers provide better electrical impedance matching of the transducer to the cable impedance, lowering the generation of heat. In another embodiment, a lensless array or a piezoelectric material shaped to provide elevation focus without a lens focus is provided to reduce the heating of the transducer 14. Reduced heating or more efficient heat dissipation allows for better penetration of acoustic energy and higher power transmissions, such as associated with color Doppler or therapeutic acoustic energy.

The transducer 14 is designed for operation within a frequency band. Typically, the frequency band is associated with transmission and reception of both imaging and therapeutic pulses having a same or similar center frequency. In alternative embodiments, the transducer 14 is associated with wide band operation, such as operating to transmit at a fundamental frequency and receive at a second or third order frequency. The imaging and therapeutic pulses may also be provided at substantially different center frequencies, such as associated with a - 6 dB down spectral bandwidth that do not overlap. Any frequency range may be used, but lower ultrasound frequencies (e.g., about or less than 2MHz center frequency) are used in one embodiment for breaking the contrast agents.

The transmitter 12 is a transmit beamformer, waveform generator, pulser or other source of electrical excitations for imaging and therapeutic transmissions. In one embodiment, the transmitter 12 is a transmit beamformer that generates waveforms for each of a plurality of channels or transducer elements, such as 128 waveforms, separately delayed and apodized for focusing transmissions along scan lines 22 within a field of view 24. Based on the delays and apodization, multiple transmissions may be sequentially scanned across substantially parallel scan lines 22 in the entire field of view 24. The field of view 24 is formed in response to the scan pattern, such as a linear, sector or Vector^{®} scan patterns. Plane wave or diverging wavefronts with or without steering are alternatively formed.

The transmitter 12 electrically connects with the transducer 14 for generating transmissions of acoustic energy or transmit pulses in response to the electrical signals from the transmitter 12. The acoustic energy transmitted includes one of imaging or therapy pulses. Imaging pulses are transmissions adapted for generating an image of the field of view 24, such as sequential transmissions of narrow beams sequentially focused along a plurality of scan lines 22. Therapy pulses include transmissions adapted for contrast agent destruction. Therapy pulses or transmissions are operable to force rupture of contrast agents. For example, higher power pulses (e.g., about or above 1.2 MI) propagate within a region of interest 26 of the field of view 24. The therapy pulses are focused along scan lines 22 within the region of interest 26. Plane or diverging wavefronts may alternatively be used.

The receive beamformer 16 generates receive beams for imaging. The receive beamformer 16 applies various delays and apodization to electrical signals received from elements of the transducer 14 and sums the signals to generate a receive beam representing a scan line 22 in response to each of the transmissions. The received echoes are responsive to the imaging transmissions. Echoes may or may not be received for imaging in response to the therapy transmissions.

The processor or detector 18 comprises one or more of an application specific integrated circuit, general processor, digital signal processor, other digital circuitry, analog circuitry, a combination thereof or other devices for detecting information from the received, beamformed signals for imaging. In one embodiment, the processor 18 comprises a B-mode or Doppler detector. For example, the amplitude of an envelope associated with the received signals is detected. As another example, a frequency shift or velocity, magnitude of a Doppler signal or energy, or variance is detected by Doppler or correlation processing for flow or tissue motion imaging. Single pulse or multiple pulse techniques for contrast agent imaging may be used, such as loss-of-correlation imaging or harmonic imaging using modulation of phase and/or amplitude and subsequent combination of received signals. U.S. Patent Nos. 6,494,841 and 6,632,177, the disclosures of which are incorporated herein by reference, teach contrast agent imaging techniques. Other contrast agent imaging techniques may be used. Other processors for one -dimensional, two -dimensional or three-dimensional imaging may be used.

A two-dimensional image is generated using any of the B-mode, Doppler or contrast agent imaging methods discussed above. The detected information from the processor 18 is provided to the display 20. An image representative of the imaging pulses is generated on the display. Various combinations or single types of images are displayed substantially simultaneously, such as one or more of a B-mode, Doppler or contrast agent image. In one embodiment, portions of a field of view 24, such as lateral edges, are shown as B-mode or Doppler images, and another portion, such as a laterally centered portion, is displayed as contrast agent image.

Using the system 10 described above, a field of view is imaged. A suspected thrombus or possible blood clot is identified on the image by the user. In one embodiment, higher power B-mode or color-flow (e.g., Doppler) imaging is used to better identify a stiffening thrombus. Contrast agents are injected. The contrast agents travel to the region of interest 26. The same type of imaging or contrast agent imaging is used to identify when sufficient contrast agents are near or in the thrombus. For example, the same system 10 and transducer 14 transmit low MI (e.g. 0.5 or less) acoustic energy for imaging contrast agents with minimal destruction.

The same system 10, including the same transmitter 12 and transducer 14, are then used to transmit therapeutic pulses. For example, therapeutic transmissions are used to destroy the contrast agents, assisting in breaking the thrombus. In one embodiment, the therapeutic pulses are the same as B-mode or color-flow pulses used for imaging. Alternatively, pulses adapted for maximizing contrast agent force from destruction are used, such as low frequency acoustic energy with a MI of about but below 1.9. A greater pulse repetition frequency may be used to increase acoustic power applied to the contrast agents. By combining the imaging and therapeutic functions in a single device, time critical procedures are expedited and treatment precision is controlled. Using a same system for both imaging and therapy also reduces the cost associated with extra equipment.

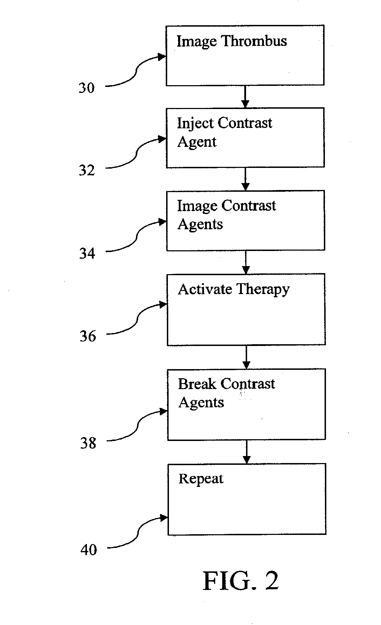

Figure 2 shows a method of one embodiment for contrast agent ultrasound imaging and therapy. The method is implemented with the system 10 of Figure 1 or a different system. Additional, different or fewer acts may be performed. For example, the repetition act 40 is not provided. The acts are performed in the order shown or a different order. For example, the thrombus is imaged in act 30 after injecting contrast agents in act 32, during the injection of act 32, at a same time as the imaging of contrast agents 34, at other times or combinations thereof. The imaging acts 30 and 34 may be ongoing while performing other acts, such as acts 36, 38 and 40, or may be discrete events that do not overlap in time with one or more other acts.

The method of Figure 2 uses the same transducer for at least one of the imaging acts 30, 34 and the breaking of contrast agents in act 38. For example, a single 64, 128, 192 or 256 element one-dimensional array is used. As another example, a multidimensional array is used. Different apertures on the same array may be used for different acts. For example, the transmit aperture for breaking contrast agents in act 38 may be sparse, wider or combinations thereof. For treatment of deep veineous thrombosis, a large transmit aperture is desired, such as a 2 cm or larger aperture. Smaller apertures may be used. The same aperture may be used for imaging and breaking contrast agents.

The transducer is hand held or mounted for use external to the patient. A mounting may provided for guided, controlled or automated sweeping or movement of the transducer. Alternatively, a wobbler array sweeps. In another alternative embodiment, the transducer is in a catheter, transesophageal, endocavity, intra-operative, or other probe for use within a patient.

In act 30, the thrombus or possible blood clot is imaged with the ultrasound transducer. B-mode, color-Doppler or another imaging mode allows detection of any thrombosis. Transmitted acoustic energy is high or low MI, such as having an MI greater than 1.0. The frequency used is within the bandwidth of the transducer. In response to the transmissions, echo signals are received using the transducer. The received signals are also responsive to the possible thrombus. Using the imaging of act 30, the location of any possible blood clot is identified. Diagnosis of a possible clot may be assisted by applying pressure with the transducer, by the operator or with another object. The transducer, operator or other object presses against the patient. A blood clot is less likely than a vein without a blood clot to collapse in response to the external pressure.

In act 32, contrast agents are injected. For example, the contrast agents are provided in the blood of a patient through intravenous infusion. Other now known or later developed techniques for introducing contrast agents adjacent to or in the thrombus may be used, such as injection with a needle or through a catheter directly in or near the possible blood clot. Any contrast agents may be used. In one embodiment, the contrast agents carry drugs or are mixed with drugs, such as drugs for assisting in disruption or weakening of the thrombus (e.g., fibrinolytic agents). In other embodiments, the contrast agents are free of any drugs. The contrast agents may be adapted for disruption, such as by having thinner or thicker walls and/or being more or less elastic.

In act 34, the contrast agents adjacent to or in the thrombus are imaged. For example, the possible blood clot continues to be imaged in act 30. As the contrast agents enter the field of view, the contrast agents are imaged with the same mode of operation in act 34 as act 30. In another example, the possible clot is imaged with a higher transmit level prior to injection and with a lower transmit level after injection. After the injection of contrast agents occurs and before or after the contrast agents enter the field of view, the same transducer images with low-MI ultrasound. The transmitted acoustic energy is maintained at about 0.5 MI or less. Greater powers may be used. The transducer receives acoustic energy in response to the transmission. The acoustic energy is also responsive to the contrast agents and the possible thrombus. Low MI and/or higher frequency imaging generate images with lesser breaking of the contrast agents than occurs in act 38. Some breakage during imaging may be acceptable. The imaging of contrast agents allows identification of when sufficient contrast agents are near or in the thrombus for treatment.

In act 36, the therapy is activated. The user or the system identifies the location of the possible blood clot. After sufficient contrast agents are detected at the location, the user activates the therapy. For example, the user presses a button on the transducer. As another example, the user depresses a foot peddle. Other user inputs, such as a button or key on a keyboard or control panel, may be used. In an alternative embodiment, the system or a processor automatically activates the therapy. The therapy can be applied to a larger or smaller region than the imaging region.

In response to the activation of act 36, mechanical contrast agent destruction therapy is applied in act 38 with the same ultrasound transducer used for imaging. Acoustic energy breaks the contrast agents at or adjacent to the possible clot. The disruption caused by the destruction of contrast agents mechanically breaks or weakens the blood clot. Disruption may also or alternatively be caused by expansion or contraction of contrast agents without breaking.

Contrast agents are destroyed or expanded by transmitting high-MI ultrasound, such as acoustic energy with an MI about or above 1.0-1.2 or more. Greater acoustic energy may provide more disruptive destruction of contrast agents, such as transmitting with an MI of about 1.9. The acoustic energy is focused at or near the possible blood clot to provide the greatest destructive power at the possible blood clot. Unfocused or weakly focused acoustic energy may be used.

Contrast agents may more likely be destroyed by pulses at lower frequencies with the same MI. For example, a center frequency of about 2.0 MHz or lower is used. Greater frequencies may be used. The duration of a transmit event for breaking contrast agents is of any length. In one embodiment, the duration is less than 50 microseconds, such as being as short as 10 to 20 microseconds. Short duration may avoid temperatures near thermal limits. Longer durations with the same or lower power may be used. The pulses may be repeated, such as repeating the transmission for a few hundreds of microseconds. Greater, lesser or no repetitions may be used. Different MI and/or thermal limits may be provided for therapy as opposed to imaging.

The transmitted acoustic pulses are square waves, sinusoids or other waveforms with or without an envelope, such as a Gaussian or rectangular envelope. In one embodiment, the pulses have a substantially uniform negative peak pressure. Since the system may not instantaneously generate the desired amplitude, the transmit waveforms are phased to begin with a positive peak pressure. By the second half of the initial cycle of the pulse, the system more likely has ramped to the desired amplitude. The negative peak pressures are more likely uniform, increasing the contrast agent destructive capabilities. In other embodiments, different phasing is provided.

The acoustic energy responsive to the therapy transmissions are not used for imaging. The imaging and breaking transmissions are interleaved, such as providing substantially continuous imaging with more sparse therapy or vice versa Frame to frame, line-to-line, group of frames, group of lines or other interleaving may be used. Alternatively, the therapy transmissions are also used for imaging. The imaging and the therapy pulses are the same or different.

In optional act 40, the breaking of contrast agents in act 38 is repeated. For example, the pulses are transmitted again to a same location. The user or a processor measurement may indicate when the blood clot is sufficiently weakened or disrupted and/or when sufficient or insufficient contrast agents are present. As another example, pulses are transmitted along different scan lines or at different angles. The acoustic energy is swept through a plane or volume. Mechanical or electrical mechanisms steer or focus the acoustic energy to different locations. Automatic or manual control of the sweep is provided. By scanning an entire blood clot in two or three dimensions, the blood clot is more likely disrupted or weakened. The region for sweeping is the same, larger or smaller than an imaging region. The imaging of acts 30 and/or 34 may track the region of sweeping. In another example, any one or more of the acts 30-38 are repeated. The treatment progress is monitored by any ultrasound imaging mode, such as B-mode two or three-dimensional imaging, color Doppler or spectral Doppler modes.

The operations of the system for automated performance of one or more of the acts of Figure 2 or for interaction to provide for manual performance are implemented with instructions by a programmed processor. The instructions for implementing the processes, methods and/or techniques discussed above are provided on computer-readable storage media or memories, such as a cache, buffer, RAM, removable media, hard drive or other computer readable storage media. Computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, filmware, micro code and the like, operating alone or in combination. Likewise, processing strategies may include multiprocessing, multitasking, parallel processing and the like. In one embodiment, the instructions are stored on a removable media device for reading by local or remote systems. In other embodiments, the instructions are stored in a remote location for transfer through a computer network or over telephone lines. In yet other embodiments, the instructions are stored within a given computer, CPU, GPU or system.

While the invention has been described above by reference to various embodiments, it should be understood that many changes and modifications can be made without departing from the scope of the invention. It is therefore intended that the foregoing detailed description be regarded as illustrative rather than limiting, and that it be understood that it is the following claims, including all equivalents, that are intended to define the spirit and scope of this invention.

## Claims

1. A method for contrast agent ultrasound imaging and therapy, the method comprising:
imaging (30, 34)a possible clot and contrast agents with an ultrasound transducer (14); and
driving (38) the contrast agents at or adjacent to the possible clot with ultrasound pulses from the same ultrasound transducer (14).

2. The method of Claim 1 wherein driving (38) comprises transmitting high-MI ultrasound.

3. The method of Claim 1 wherein driving (38) comprises transmitting at least one pulse at a frequency about 2 MHz or below with a duration less than 50 microseconds.

4. The method of Claim 1 wherein driving (38) comprises transmitting pulses with a positive pressure occurring before a negative pressure in an initial cycle.

5. The method of Claim 1 further comprising:
activating (36) the driving act with a button on the ultrasound transducer (14) or with a foot pedal.

6. The method of Claim 1 wherein imaging (30, 34) comprises imaging (34) with low-MI ultrasound.

7. The method of Claim 1 wherein imaging (30, 34) comprises imaging (30) the possible clot with a higher transmit level prior to injection of the contrast agents and imaging (34) with a lower transmit level after injection of the contrast agent.

8. The method of Claim 1 wherein imaging (30, 34) comprises generating images with lesser breaking of the contrast agents than the driving (38).

9. The method of Claim 1 further comprising:
sweeping the possible clot during the driving (38).

10. The method of Claim 1 further comprising:
repeating (40) the imaging and driving until the possible clot is substantially not visible in the imaging (30, 34).

11. The method of Claim 1 wherein imaging (30, 34) comprises imaging a thrombosis.

12. The method of Claim 1 wherein driving (38) comprises releasing a drug from the contrast agents.

13. The method of Claim 1 further comprising:
monitoring treatment.

14. In a computer readable storage medium having stored therein data representing instructions executable by a programmed processor for contrast agent ultrasound imaging and therapy, the storage medium comprising instructions for:
using one transducer (14) to image (30, 34) a blood clot and contrast agents and to mechanically break or weaken (38) the blood clot with disruption by at least some of the contrast agents.

15. The instructions of Claim 14 wherein mechanically breaking or weakening (38) the blood clot comprises transmitting ultrasound energy with a mechanical index above 1.2.

16. The instructions of Claim 14 wherein mechanically breaking or weakening (38) the blood clot comprises transmitting at least one pulse at a center frequency about 2 MHz or below with a duration less than 50 microseconds and a positive pressure occurring before a negative pressure in an initial cycle of the at least one pulse.

17. The instructions of Claim 14 further comprising:
activating (36) the breaking or weakening act in response to a button on the ultrasound transducer (14) or with a foot pedal.

18. The instructions of Claim 14 wherein imaging (30, 34) comprises imaging (30) the blood clot with a higher transmit level prior to injection of the contrast agents, imaging (34) with a lower transmit level after injection of the contrast agent, and interleaving the imaging (34) with the breaking or weakening (38).

19. The instructions of Claim 14 further comprising:
repeating (40) the imaging and breaking or weakening (38) until the blood clot is substantially not visible in the imaging (30, 34), the imaging (30, 34) comprising, at least in part, monitoring a treatment.

20. A method for contrast agent ultrasound imaging and therapy, the method comprising:
first transmitting acoustic energy with an ultrasound transducer (14) to a thrombosis, the transmitting being about 0.5 MI or less;
receiving, with the ultrasound transducer (14), acoustic energy in response to the first transmitting, the received acoustic energy responsive to contrast agents and the thrombosis;
second transmitting acoustic energy, with the ultrasound transducer (14), the transmitting being about 1.0 MI or more and operable to alter the contrast agents associated with the thrombosis.

21. The method of Claim 20 wherein acoustic energy responsive to the second transmitting is not used for imaging.

22. The method of Claim 20 wherein first transmitting and receiving comprise imaging (30, 34) with the ultrasound transducer (14) and wherein second transmitting comprises applying (38) mechanical contrast agent destruction therapy with the same ultrasound transducer (14).

23. The method of Claim 20 wherein second transmitting comprises destroying (38) the contrast agents, the contrast agents being mixed with or carrying a drug.
